# EUROPÄISCHE PATENTANMELDUNG

(11) **EP 2 522 223 A1**
(43) Veröffentlichungstag der Anmeldung: **14.11.2012**
(21) Anmeldenummer: 12005697.3
(22) Anmeldetag: 14.11.2009
(51) Int. Cl.: A01N 59/16, A01N 59/20, A61K 31/375, A61K 33/24, A61K 33/30, A61K 33/34, A01N 25/30, A01N 33/12, A01N 37/36, A01N 43/08, A01P 1/00

(54) **Kombinierte Desinfektions- und Dekontaminationsmittel mit erhöhter Wirksamkeit**

(30) Priorität: 18.12.2008 DE 102008064481
(62) Teilanmeldung aus: 09756674.9
(71) Anmelder: Bode Chemie GmbH, 22525 Hamburg (DE); multiBIND biotec GmbH, 51105 Köln (DE)
(72) Erfinder: Die Erfindernennung liegt noch nicht vor
(74) Vertreter: Kossak, Sabine

(57) **Zusammenfassung**

Gegenstand der Erfindung ist ein neues, kombiniertes Desinfektions- und Dekontaminationsmittel, das mindestens ein Vitamin, mindestens ein Metallion, mindestens eine oberflächenaktive Verbindung und mindestens eine quaternäre Ammoniumverbindung als antimikrobiellen Wirkstoff enthält. Überraschenderweise zeigt das erfindungsgemäße Mittel neben einer verbesserten Desinfektionswirkung einen nahezu vollständigen Nukleinsäure-Abbau. Das Mittel kann erfolgreich als kombiniertes Dekontaminations- und Desinfektionsmittel für Haut, Schleimhäute, Hände, Wunden und/oder Haare bzw. Instrumenten und Flächen aller Art eingesetzt werden.

## Beschreibung

Gegenstand der Erfindung ist ein Desinfektions- und Dekontaminationsmittel, enthaltend mindestens ein Vitamin, mindestens ein Metallion, mindestens eine oberflächenaktive Verbindung und einen antimikrobiellen Wirkstoff aus der Gruppe der quaternären Ammoniumverbindungen.

Im medizinischen Bereich, bei der Produktion von Lebensmitteln, in Versuchslaboratorien und anderen sensiblen Bereichen ist es notwendig, Mikroorganismen abzutöten und möglichst vollständig zu entfernen. Dabei ist es zum einen bekannt, die Mikroorganismen in Form einer Desinfektion zu bekämpfen. Unter Desinfektion wird allgemein die effektive, irreversible Inaktivierung, Abtötung oder Entfernung von Mikroorganismen verstanden. Dabei zählen zu den Mikroorganismen Bakterien, Mykobakterien, Pilze, Hefen, Sporen, Prionen, Mykoplasmen und/oder Viren. Diese werden bei der Desinfektion von belebten und/oder unbelebten Flächen, Geweben oder Räumen entfernt.

In einigen Bereichen hat sich gezeigt, dass eine Desinfektion allein nicht ausreichend ist, da auch die abgetöteten Mikroorganismen bzw. einzelne Nukleinsäure-Moleküle wie DNA und RNA der Mikroorganismen oder Proteine oder andere Rückstände direkt oder über Steigerung der Infektiösität oder Resistenz anderer Mikroorganismen zu weiteren Infektionen führen können. Zur vollständigen Beseitigung der Kontamination mit Proteinen oder Nukleinsäuren werden daher Dekontaminationsmittel eingesetzt. Die DNA/RNA-Dekontaminationsmittel sollen dabei zu einer wirksamen Dekontamination einen möglichst vollständigen Nukleinsäure-Abbau bewirken.

Bei einer vollständigen Desinfektion und Dekontamination einer belebten oder unbelebten Oberfläche ist es daher notwendig, zwei Mittel anzuwenden, nämlich ein Desinfektionsmittel zur Abtötung der Mikroorganismen und ein Dekontaminationsmittel zur Beseitigung der verbleibenden Restkontamination durch Nukleinsäure-Moleküle, Proteine oder andere Rückstände. Die Anwendung eines Dekontaminationsmittels sichert die effiziente Dekontamination, indem die freien Nukleinsäure-Moleküle modifiziert, denaturiert oder degradiert werden. Die bekannten Dekontaminationsmittel haben den Nachteil, dass sie häufig chemisch aggressiv und gesundheitlich nicht unbedenklich sind. Bei der Verwendung von aggressiven Substanzen greifen diese beispielsweise unbelebte Oberflächen an und führen zu einer dauerhaften Beschädigung oder Verfärbung. Auf belebten Oberflächen wie Hände, Haut, Schleimhaut sind viele aggressive Dekontaminationsmittel aus gesundheitlichen Gründen überhaupt nicht einsetzbar.

Ein weniger aggressives Dekontaminationsmittel ist beispielsweise aus der DE 10 2005 020 327 A1 bekannt. Diese Dekontaminationslösung aus einem Vitamin, einem Metallion und einer oberflächenaktiven Verbindung zeigt einen guten Nukleinsäure-Abbau bei gleichzeitiger Schonung der Oberflächen. Allerdings konnte beispielsweise keine ausreichende Wirksamkeit in den üblichen Anwendungskonzentrationen und Einwirkzeiten gegen Candida albicans festgestellt werden.

Aufgabe der vorliegenden Erfindung ist es daher, ein neues Kombinationsmittel bereit zu stellen, das erstmalig eine gleichzeitige Desinfektion und Dekontamination von belebten und unbelebten Oberflächen bewirkt. Das Mittel soll schonend zu Oberflächen sein, d.h. eine gute Materialverträglichkeit bzw. Haut- und Schleimhautverträglichkeit aufweisen. Das Mittel soll neben einer zuverlässigen Desinfektion einen möglichst vollständigen Nukleinsäure-Abbau zeigen.

Die Aufgabe wird erfindungsgemäß gelöst durch ein Desinfektions- und Dekontaminationsmittel bestehend aus mindestens einem Vitamin, mindestens einem Metallion, mindestens einer oberflächenaktiven Verbindung und mindestens einem antimikrobiellen Wirkstoff aus der Gruppe der quaternären Ammoniumverbindungen, gegebenenfalls mindestens einem weiteren antimikrobiellen Wirkstoff, ausgewählt aus der Gruppe bestehend aus:
- organischen und anorganischen Säuren oder deren Salzen, Estern oder Amiden,
- aliphatischen oder aromatischen Mono- oder Dialdehyden,
- Peroxiden oder Persäuren,
- Guanidinen,
- Pyridinen oder Pyrimidinen,
- amphoteren Wirkstoffen ausgewählt aus der Gruppe der
   - Reaktionsprodukte aus n-C10-C16-Alkyltrimethylendiamin und Chloressigsäure (Ampholyt 30)
   - [2-[[2-[(2-Carboxyethyl)(2-hydroxyethyl)amino]ethyl]amino]-2-oxoethyl]kokosalkyldimethylamine, deren Hydroxide oder deren "Innere Salze" (Rewoteric QAM 50) oder
   - N-[3-(Dodecylamino)propyl]glycin Hydrochlorid (Amphionic SFB),
   - aliphatischen sekundären oder tertiären Aminen oder
   - einer Mischung hiervon,
   - Hilfsstoffen und
   - gegebenenfalls Wasser,
   - wobei sich die Komponenten zu 100 Gew.-% ergänzen.

Weitere Ausführungsformen sind Gegenstand der Unteransprüche oder nachfolgend beschrieben.

Als antimikrobieller Wirkstoff ist mindestens eine quaternäre Ammoniumverbindung enthalten. Geeignete quaternäre Ammoniumverbindungen sind beispielsweise Didecyldimethylammoniumchlorid (DDAC), Benzalkoniumchlorid (BAC), Benzalkoniumbromid, Benzoxoniumchlorid, Dioctyldimethylammoniumchlorid, N,N-Dialkyl-N,N-dimethylammoniumcarbonat, quaternäre Ammoniumiodide, quaternäre Ammoniumverbindungen des Typs Dialkyldimethylammoniumchloride oder -bromide oder-methylsulfate/DDAC (mit Alkyl aus C6 bis C18, gesättigt oder ungesättigt, und Talgalkyl, Kokosalkyl und Sojaalkyl), Mecetroniumetilsulfat (MES), Dimethyldecyloxethylammoniumpropionat, Cetrimid, Cetrimoniumbromid, Cetylpyridiniumchlorid oder Undecylenamidopropyltrimoniummethosulfat. Besonders bevorzugt werden als antimikrobieller Wirkstoff Didecyldimethylammoniumchlorid (DDAC), Benzalkoniumchlorid (BAC) oder Mecetroniumetilsulfat (MES) verwendet. Diese werden bevorzugt in einer Konzentration von 0,01 - 25 Gew.-%, besonders bevorzugt 0,01 - 20 Gew.-% und insbesondere 0,01 - 15 Gew.-% eingesetzt. Die quaternären Ammoniumverbindungen werden weiterhin bevorzugt in Konzentrationen von 0,01 - 10 Gew.-%, besonders bevorzugt 0,01 - 5,0 Gew.-% und insbesondere 0,01 - 2 Gew.-% eingesetzt.

In einer weiteren Ausführungsform ist mindestens ein weiterer antimikrobieller Wirkstoff enthalten, wobei dieser im erfindungsgemäßen Desinfektions- und Dekontaminationsmittel eingesetzte antimikrobielle Wirkstoff ein Aldehyd ist, das ausgewählt ist aus der Gruppe der aliphatischen oder aromatischen Mono- oder Dialdehyde. Geeignete Mono- und Dialdehyde sind insbesondere Glutardialdehyd, Succindialdehyd, ortho-Phthalaldehyd, Glyoxal, Acrolein, Piperonal, Formaldehyd, Formaldehyabspalter und Abspalter von Dialdehyden. Formaldehydabspalter sind z.B. (Ethylendioxy)dimethanol, Tetrahydro-1,3,4,6-tetrakis(hydroxymethyl)imidazo[4,5-d]imidazol-2,5(1 H,3H)-dion, Imidazolidinylharnstoff, 1,3-Bis(hydroxymethyl)harnstoff, Diazolidinylharnstoff, Triazine, Paraformaldehyd, Hexamethylentetraamin, Quaternium^{®} 15 oder Dimetyloldimethylhydantoin. Bevorzugt werden die Monoaldehyde und/oder Dialdehyde in Konzentrationen von 0,01 - 15 Gew.-%, besonders bevorzugt 0,1 - 10 Gew.-% und insbesondere 0,01 - 5,0 Gew.-% eingesetzt, insbesondere 0,025 - 2,5 Gew.-%.

Der ggf. mindestens eine weitere antimikrobielle Wirkstoff ist in einer weiteren Ausführungsform eine aliphatische oder aromatische organische Säure oder eine anorganische Säure oder deren Salz, Ester oder Amid. Geeignete organische Säuren sind beispielsweise Ameisensäure, Essigsäure, Bromessigsäure, Glykolsäure, Propionsäure, Glyoxylsäure, Milchsäure, Zitronensäure, Weinsäure, Malonsäure, Maleinsäure, Fumarsäure, Pyrrolidoncarbonsäure, Sorbinsäure, Undecylensäure, Undecinsäure, Benzoesäure, Hydroxybenzoesäure, Salicylsäure, Dehydracetsäure, 4-Hydroxybenzoesäureester (Parabene), Dimethylcarbonat, Chloracetamid, 2-Chlor-N-(hydroxymethyl)acetamid und/oder Salicylanilid, bevorzugt Glykolsäure, Milchsäure, Zitronensäure, Pyrrolidoncarbonsäure, Sorbinsäure, Undecylensäure, Hydroxybenzoesäure, Salicylsäure, Dehydracetsäure und/oder 4-Hydroxybenzoesäurester (Parabene). Die organische Säure wird bevorzugt in einer Konzentration von 0,01 - 10 Gew.-% oder 0,01 - 5,0 Gew.-%, besonders bevorzugt 0,01 - 3,0 Gew.-% und insbesondere 0,01 - 2,5 Gew.-% oder 0,025 - 2,5 Gew.-% eingesetzt. Geeignete anorganische Säuren sind beispielsweise Phosphorsäure, Schwefelsäure, Salzsäure, Borsäure, schwefelige Säure, Salpetersäure oder Kohlensäure. Die anorganischen Säuren werden bevorzugt in denselben Konzentrationen wie die organischen Säuren eingesetzt.

In einer weiteren Ausführungsform ist der weitere antimikrobielle Wirkstoff ein Peroxid oder eine Persäure, wobei bevorzugte Peroxide oder Persäuren ausgewählt sind aus der Gruppe Wasserstoffperoxid, Benzoylperoxid, Peressigsäure, Natriumperborat, Magnesium-Monoperoxyphthalat oder Magnesium-Monoperoxyphthalat Hexahydrat (MMPP), Pentakalium-bis(peroxymonosulfat)-bis(sulfat), Dinatriumperoxodisulfat/Natriumpersulfat, Dikaliumperoxodisulfat, Dinatriumcarbonat mit Hydrogenperoxid, 2-Butanon-peroxid, tert-Butylhydroperoxid, Peroxyoctansäure, Persäureester oder Reaktionsprodukt aus Dimethyladipat, Dimethylglutarat, Dimethylsuccinat mit Wasserstoffperoxid, z.B. Perestane^{®} (Solvay), und/oder Phthalimid-Peroxy-Capronsäure, 6-(Phthalimid)peroxyhexansäure, bevorzugt Wasserstoffperoxid oder Magnesium-Monoperoxyphthalat Hexahydrat (MMPP). Die Peroxide werden bevorzugt in einer Konzentration von 0,01 - 10 Gew.-% oder 0,01 - 5,0 Gew.-% verwendet, weiter bevorzugt von 0,01 - 2,0 Gew.-%, besonders bevorzugt 0,01 - 1,0 Gew.-% und insbesondere 0,01 - 0,5 Gew.-% eingesetzt.

In einer weiteren Ausführungsform ist als weiterer antimikrobieller Wirkstoff eine Guanidin-, Pyridin- oder Pyrimidinverbindung enthalten, wie beispielsweise PHMG, Dipyrithion, Pyrithion-Zink oder Octenidinsalze wie z.B. das Dihydrochlorid. Diese Wirkstoffe werden beispielsweise in einer Konzentration von 0,01 - 20 Gew.-%, bevorzugt 0,01 - 10 Gew.-%, sowie 0,01 - 5,0 Gew.-% eingesetzt, wobei bevorzugt Konzentrationen von 0,01 -2,0 Gew.-%, besonders bevorzugt 0,01 - 1,5 Gew.-% und insbesondere 0,05 - 1,0 Gew.-% oder 0,05 - 0,5 Gew.-% eingesetzt werden.

In einer weiteren Ausführungsform ist als weiterer antimikrobieller Wirkstoff ein aliphatisches sekundäres oder tertiäres Amin enthalten, wie beispielsweise ein N-(3-Aminopropyl)-N-dodecylpropan-1,3-diamin, N-Dodecylpropan-1,3-diamin oder ein Umsetzungsprodukt von L-Glutaminsäure und Cocospropylen-1,3-Diamin, wie es unter der Marke Glucoprotamin^{®} verkauft wird. Die aliphatischen Amine werden bevorzugt in einer Konzentration von 0,01 - 25 Gew.-%, bevorzugt 0,01 - 20 Gew.-%, sowie 0,01 - 15 Gew.-% eingesetzt, wobei Konzentrationen von 0,01 - 10 Gew.-% bevorzugt sind, besonders bevorzugt werden 0,01 - 5,0 Gew.-% und insbesondere 0,01 - 2,0 Gew.-% eingesetzt.

In einer weiteren Ausführungsform ist als weiterer antimikrobieller Wirkstoff ein amphoterer Wirkstoff enthalten, ausgewählt aus der Gruppe bestehend aus
- Reaktionsprodukten aus n-C10-C16-Alkyltrimethylendiamin und Chloressigsäure (Ampholyt 30) oder
- Reaktionsprodukten aus 1,3-Propandiamin, C10-C16-Alkylderivat und Chloressigsäure oder
- [2-[[2-[(2-Carboxyethyl)(2-hydroxyethyl)amino]ethyl]amino]-2-oxoethyl]kokosalkyldimethylaminen, deren Hydroxiden oder deren "Inneren Salzen" (Rewoteric QAM 50) oder
- Cocobetainamidoamphopropion oder
- N-[3-(Dodecylamino)propyl]glycin Hydrochlorid (Amphionic SFB) oder
- Alkylaminocarboxylat.

Die amphotere Verbindung wird bevorzugt in einer Konzentration von 0,01 - 25 Gew.-%, besonders bevorzugt 0,01 - 20 Gew.-% und insbesondere 0,01 - 15 Gew.-%, 0,01 - 10 Gew.-%, besonders bevorzugt 0,01 - 5,0 Gew.-% und insbesondere 0,01 - 2,0 Gew.-% eingesetzt.

Das im erfindungsgemäßen Desinfektionsmittel eingesetzte Vitamin, dessen Salz oder saures Derivat ist bevorzugt ein Vitamin mit der Eigenschaft von Antioxidantien. Geeignete Vitamine sind insbesondere Vitamin A, Vitamin C (D-Ascorbinsäure), B und E. Insbesondere bevorzugte Vitamine sind die wasserlöslichen Vitamine B und C. Das Vitamin ist in Mengen von 0,1 mM bis 1000 mM, besonders bevorzugt 0,1 mM bis 500 mM, weiterhin bevorzugt 0,1 mM bis 300 mM und insbesondere 0,1 mM bis 100 mM im erfindungsgemäßen Desinfektions- und Dekontaminationsmittel enthalten.

Als Metallion wird im erfindungsgemäßen Desinfektions- und Dekontaminationsmittel bevorzugt ein Ion eines Metalls der vierten Periode oder Ion eines Metalls der Nebengruppen I., II. oder VIII. des periodischen Systems der Elemente eingesetzt. Besonders bevorzugte Metallionen sind Eisen, Kobalt, Zink, Kupfer und Silber. Das Metallion ist in Mengen von 0,01 mM bis 100 mM, vorzugsweise 0,01 mM bis 50 mM, insbesondere 0,01 mM bis 30 mM, besonders bevorzugt 0,01 mM bis 10 mM im erfindungsgemäßen Mittel enthalten.

Das erfindungsgemäße Desinfektions- und Dekontaminationsmittel enthält bevorzugt als oberflächenaktive Substanz eine Verbindung, ausgewählt aus der Gruppe bestehend aus anionischen, nichtionischen, amphoteren oder kationischen Tensiden oder eine Mischung hiervon. Die oberflächenaktive Substanz ist dabei in Mengen von 0,01 Gew.-% bis 35 Gew.-%, vorzugsweise 0,01 Gew.-% bis 30 Gew.-%, insbesondere 0,01 Gew.-% bis 20 Gew.-%, ganz besonders bevorzugt 0,025 Gew.-% bis 15 Gew.-% in der Gesamtlösung enthalten. Insbesondere können Alkylethersulfate, Alkyl- und/oder Arylsulfonate, Alkylsulfate, Amphotenside, Betaine, Alkylamidoalkylamine, alkylsubstituierte Aminosäuren und/oder Iminosäuren, acylierte Aminosäuren und/oder Amphotensidkombinationen wie Fettalkoholethoxylate, Fettalkoholpropoxylate, Alkylphenolpolyglycolether, verzweigtkettige Alkylethoxylate, Fettsäureethoxylate, Alkylpolyglucoside, Saccharoseester, Sorbitanester, Aminoxide eingesetzt werden. Grundsätzlich sind alle Tenside geeignet. Erfindungsgemäß bevorzugt sind anionische und nichtionische Tenside.

Alle Konzentrationsangaben beziehen sich jeweils auf die fertige Anwendungslösung bzw. Applikationsform. Beim Einsatz unverdünnter Lösungen geben die Konzentrationen somit die Menge in der unverdünnten Lösung an, beim Einsatz von Konzentraten geben die Konzentrationen die Menge in der aus dem Konzentrat hergestellten Anwendungslösung wieder. Entsprechendes gilt für feste und halbfeste Produkte, die zur Anwendung in Lösemitteln gelöst werden.

Das erfindungsgemäße Desinfektions- und Dekontaminationsmittel kann in flüssiger, halbfester oder fester Form eingesetzt werden, wobei folgende Applikationsformen bevorzugt sind: Lösung, Suspension, Emulsion, Paste, Gel, Schaum, Tablette, Pulver, Granulat, Tücher, selbstauflösende Beutel und/oder Zweikomponentensysteme.

Das erfindungsgemäß Desinfektions- und Dekontaminationsmittel kann in Form von flüssigen Konzentraten vorliegen, so dass eine Gebrauchslösung jeweils frisch angesetzt wird. Die flüssigen Konzentrate werden dabei vorzugsweise mit Wasser zu einer Anwendungslösung verdünnt.

Die eingesetzten Hilfsstoffe sind vorzugsweise Farbstoffe, Konservierungsmittel, Emulgatoren, Antioxidantien, Pflegestoffe, Konservierungsmittel, Konsistenzgeber, Lösungsmittel, Verdickungsmittel, Parfüm, Korrosionsinhibitoren, Komplexbildner und/oder pH-Regulatoren.

Geeignete Verdickungsmittel sind beispielsweise, ohne darauf beschränkt zu sein, Polymere der Acrylsäure oder deren Derivate, wie z. B. Polyacrylsäure, Acrylamid-Copolymer, Acrylamid/Natriumacrylat-Copolymer, Acrylat-Copolymer, Ammoniumacrylat-Copolymer, Natriumacrylatpolymer, quervernetzte Acrylatpolymere, wie z. B. Acrylates/C10-30 Alkyl Acrylate Crosspolymer, Carbomer, quervernetztes Methylmethacrylat-Copolymer, natürliche Polymere und deren Derivate, wie z. B. Ammoniumalginat, Carboxymethylcellulose, Carboxymethyldextran, Ethylcellulose, Guarhydroxypropyltrimoniumchlorid, Hydroxyethylcellulose, Hydroxyethylchitosan, Hydroxypropylstärke, Hydroxypropylstärkephosphat, Hydroxyethylmethylcellulose, Silikonpolymere, wie z. B. quervernetztes Divinyldimethicon/Dimethicon-Copolymer, Polyglykole, wie z. B. Polyethylenglykol, Polypropylenglykol, Polyethylen-co-propylenglykol mit unterschiedlichen Molekulargewichten, Polyvinylalkohole unterschiedlichen Molekulargewichts, quervernetztes Copolymer aus Methylvinylether/Maleinsäureanhydrid/Decadien-Einheiten (INCI: PVM/MA/Decadiene Crosspolymer), Polyvinylpyrrolidon, u.a.

Das erfindungsgemäße Mittel hat einen pH-Wert zwischen 0,5 bis 8,5, insbesondere von 1 bis 7 und vorzugsweise von 2 bis 6 bzw. 2 bis 4,5. Zur pH-Wert-Einstellung kann in einer Variante ein Puffersystem eingesetzt werden. Geeignete Puffersysteme sind beispielsweise ein Puffersystem mit Carbonaten und Derivaten der Bernsteinsäure, vorzugsweise jeweils in einer Konzentration von 1 mM bis 500 mM. Bei der Verwendung dieses Puffersystems in dem erfindungsgemäßen Mittel kann der pH-Wert der Lösung, der sich aufgrund der gelösten Komponenten, insbesondere der sauren Vitamine, im stark sauren Bereich befindet, leicht bis beispielsweise in den neutralen oder schwach basischen Bereich angehoben werden, ohne dass die gelösten Metallionen ausfallen. Geeignete Systeme sind u. a. Borat-, Oxalat-, Phthalat-, Glycin-, Tartrat-, Phosphat-, Carbonat-, Citrat-, Acetatpuffer.

Das erfindungsgemäße Mittel wird in einer Ausführungsform zur Dekontamination und Desinfektion von belebten Oberflächen, vorzugsweise zur Desinfektion und Dekontamination von Haut, Schleimhaut, Wunden, Haaren und/oder Händen verwendet. In einer weiteren Ausführungsform wird das Mittel zur Desinfektion und Dekontamination unbelebter Oberflächen, vorzugsweise zur Desinfektion von Instrumenten und/oder Oberflächen verwendet.

Überraschenderweise konnte festgestellt werden, dass das erfindungsgemäße Mittel einen vollständigen und zuverlässigen Nukleinsäure-Abbau, d.h. eine vollständige Denaturierung und Entfernung von Nukleinsäure-Molekülen, leistet bei gleichzeitiger guter Desinfektionswirkung. Die Kombination des antimikrobiellen Wirkstoffs mit der Kombination aus Vitamin, Metallion und oberflächenaktiver Verbindung zeigt dabei einen unerwarteten Synergismus und führt zu einer deutlich verbesserten Desinfektionswirkung gegenüber den Einzelkomponenten. Auch konnte die Wirkung gegen Viren und Bakterien durch Kombination von Metallsalz, Vitamin, oberflächenaktiver Verbindung und antimikrobiellen Wirkstoff signifikant gesteigert werden.

Das erfindungsgemäße Desinfektions- und Dekontaminationsmittel zeigt zudem eine deutlich bessere Verträglichkeit, sowohl hinsichtlich Material als auch Haut, als herkömmliche Desinfektionsmittel. Die bessere Verträglichkeit wird unter anderem dadurch bedingt, dass durch den besonderen Synergismus, der in der Kombination der vier Wirkstoffe entsteht, eine geringere Konzentration des antimikrobiellen Wirkstoffs als bei üblichen Desinfektionsmitteln eingesetzt werden kann. Hieraus ergibt sich sowohl die bessere Material- und Hautverträglichkeit, als auch eine bessere Umweltverträglichkeit. Der Einsatz des antimikrobiellen Wirkstoffs in einer geringeren Konzentration ist somit ein wesentlicher Vorteil der synergistischen Kombination.

Das erfindungsgemäße Desinfektions- und Dekontaminationsmittel kann zudem zum Abbau von Biofilmen eingesetzt werden. Neuere Untersuchungen haben ergeben, dass medizinische Instrumente oder Implantate mikrobiell kontaminiert sind, wobei die Kontamination in Form von bakteriellen Biofilmen erfolgt. Überraschenderweise hat sich gezeigt, dass das erfindungsgemäße Desinfektions- und Dekontaminationsmittel nicht nur isolierte Bakterien beseitigt, sondern auch die in Biofilmen vorkommenden Bakterien und Pilze. Die Prüfung des Biofilmabbaus wurde mit angezüchtetem Biofilm (auf Silikonoberfläche) von Pseudomonas aeruginosa durchgeführt. Die Einwirkzeit betrug 30 min bei 25°C.

Die Verwendung des erfindungsgemäßen Desinfektions- und Dekontaminationsmittels führt zu einem zuverlässigen Abbau der Biofilme.

Die erfindungsgemäßen Vorteile sollen anhand der folgenden Beispiele näher erläutert werden.

### 1. Fungizide Wirksamkeit

Die Tabellen 1a und 1b zeigen, dass bei der Verwendung des erfindungsgemäßen Mittels als Flächen- und Instrumentendesinfektions- und -dekontaminationsmittel bereits nach einer kurzen Einwirkzeit von 5 min eine deutlichere Reduktion erreicht werden kann, als der Wirkstoff alleine oder als herkömmliche Dekontaminationsmittel alleine zeigen.

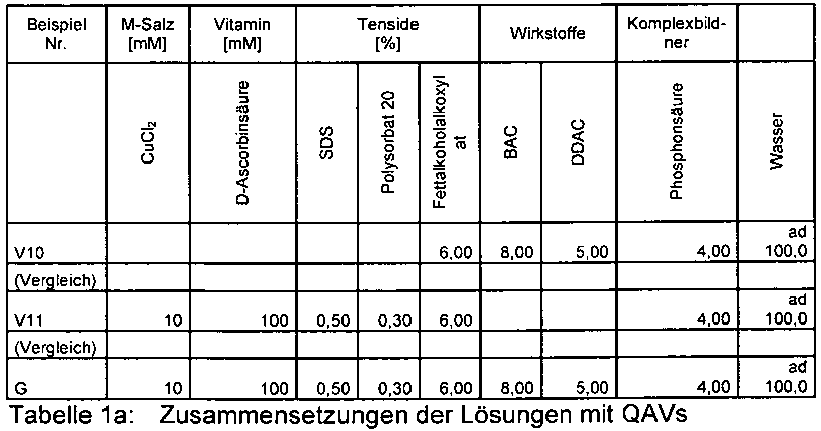

**Tabelle 1 b: Reduktionsfaktoren eines getesteten erfindungsgemäßen Mittels mit QAV.**

| | | | Candida albicans |
|---|---|---|---|
| Beispiel Nr. | pH | Anwendungskonz. [%] | 5 min |
| V10 (Vergleich) | 3 | 0,5 | 2,49 |
| | | 1 | 2,72 |
| | | 2 | 2,99 |
| | | 4 | 3,65 |
| V11 (Vergleich) | 3 | 0,5 | 0,18 |
| | | 1 | 0,27 |
| | | 2 | 0,32 |
| | | 4 | 0,36 |
| G | 3 | 0,5 | 1,59 |
| | | 1 | 3,26 |
| | | 2 | 4,97 |
| | | 4 | 4,97 |

## Patentansprüche

1. Desinfektions- und Dekontaminationsmittel bestehend aus
- mindestens einem Vitamin,
- mindestens einem Metallion,
- mindestens einer oberflächenaktiven Verbindung und
- mindestens einem antimikrobiellen Wirkstoff aus der Gruppe der quaternären Ammoniumverbindungen,
- gegebenenfalls mindestens einem weiteren antimikrobiellen Wirkstoff, ausgewählt aus der Gruppe bestehend aus
- organischen und anorganischen Säuren oder deren Salzen, Estern, Amiden,
- aliphatischen oder aromatischen Monoaldehyden oder Dialdehyden,
- Peroxiden oder Persäuren,
- Guanidinen,
- Pyridinen oder Pyrimidinen,
- amphoteren Wirkstoffen ausgewählt aus der Gruppe der:
- n-C10-C16-Alkyltrimethylendiamine, Reaktionsprodukte aus Chloressigsäure (Ampholyt 30),
- [2-[[2-[(2-Carboxyethyl)(2-hydroxyethyl)amino]ethyl]amino]-2-oxoethyl]kokosalkyldimethylaminen, deren Hydroxiden oder deren "Inneren Salzen" oder
- N-[3-(Dodecylamino)propyl]glycin Hydrochlorid
- aliphatischen sekundären oder tertiären Aminen oder
- einer Mischung hiervon,
- Hilfsstoffen und
- gegebenenfalls Wasser,
wobei sich die Komponenten zu 100 Gew.-% ergänzen.

2. Mittel gemäß Anspruch 1, **dadurch gekennzeichnet, dass** die quaternäre Ammoniumverbindung ausgewählt ist aus der Gruppe, bestehend aus Didecyldimethylammoniumchlorid (DDAC), Benzalkoniumchlorid (BAC), Benzalkoniumbromid, Benzoxoniumchlorid, Dioctyldimethylammoniumchlorid, N,N-Dialkyl-N,N-dimethylammoniumcarbonat, quaternären Ammoniumiodiden, quaternären Ammoniumverbindungen des Typs Dialkyldimethylammonium-Chlorid, -Bromid oder -Methylsulfat/DDAC mit Alkyl = C6-C18, gesättigt und ungesättigt, und Talgalkyl, Kokosalkyl und Soyaalkyl, Mecetroniumetilsulfat (MES), Dimethyldecyloxethylammoniumpropionat, Cetrimid, Cetrimoniumbromid, Cetylpyridiniumchlorid und/oder Undecylenamidopropyltrimoniummethosulfat.

3. Mittel gemäß einem der vorhergehenden Ansprüche, wobei der weitere antimikrobielle Wirkstoff ein Mono- oder Dialdehyd ist, **dadurch gekennzeichnet, dass** der Aldehyd ausgewählt ist aus der Gruppe Glutardialdehyd, Succindialdehyd, ortho-Phthalaldehyd, Glyoxal, Acrolein, Piperonal, Formaldehyd, Formaldehyabspalter und/oder Abspalter von Dialdehyden.

4. Mittel gemäß einem der vorhergehenden Ansprüche, wobei der weitere antimikrobielle Wirkstoff eine aliphatische oder aromatische organische Säure oder eine anorganische Säure oder deren Salz, Ester oder Amid ist, **dadurch gekennzeichnet, dass** dieser antimikrobielle Wirkstoff ausgewählt ist aus der Gruppe bestehend aus Ameisensäure, Essigsäure, Bromessigsäure, Glykolsäure, Propionsäure, Glyoxylsäure, Milchsäure, Zitronensäure, Weinsäure, Malonsäure, Maleinsäure, Fumarsäure, Pyrrolidoncarbonsäure, Sorbinsäure, Undecylensäure, Undecinsäure, Benzoesäure, Hydroxybenzoesäure, Salicylsäure, Dehydracetsäure, 4-Hydroxybenzoesäureester, Dimethylcarbonat, Chloracetamid, 2-Chlor-N-(hydroxymethyl)acetamid, Salicylanilid, Phosphorsäure, Schwefelsäure, Salzsäure, Borsäure, schwefelige Säure, Salpetersäure und/oder Kohlensäure.

5. Mittel gemäß einem der vorhergehenden Ansprüche, wobei der weitere antimikrobielle Wirkstoff ein Peroxid oder eine Persäure ist, **dadurch gekennzeichnet, dass** das Peroxid oder die Persäure ausgewählt ist aus der Gruppe Wasserstoffperoxid, Benzoylperoxid, Peressigsäure, Natriumperborat, Magnesium-Monoperoxyphthalat oder Magnesium-Monoperoxyphthalat Hexahydrat (MMPP), Pentakalium-bis(peroxymonosulfat)-bis(sulfat), Dinatriumperoxodisulfat/Natriumpersulfat, Dikaliumperoxodisulfat, Dinatriumcarbonat mit Hydrogenperoxid, 2-Butanon-peroxid, tert-Butylhydroperoxid, Peroxyoctansäure, Persäureester oder Reaktionsprodukt aus Dimethyladipat, Dimethylglutarat, Dimethylsuccinat mit Wasserstoffperoxid und/oder Phthalimid-Peroxy-Capronsäure, 6-(Phthalimid)peroxyhexansäure und vorzugsweise ausgewählt ist aus Wasserstoffperoxid, Magnesium-Monoperoxyphthalat Hexahydrat (MMPP) oder deren Mischungen.

6. Mittel gemäß einem der vorhergehenden Ansprüche, wobei der weitere antimikrobielle Wirkstoff ein Guanidin-, Pyridin- oder Pyrimidin-Derivat ist, vorzugsweise PHMG oder ein Octenidinsalz.

7. Mittel gemäß einem der vorhergehenden Ansprüche, **dadurch gekennzeichnet, dass** die quaternäre Ammoniumverbindung ausgewählt ist aus der Gruppe, bestehend aus Didecyldimethylammoniumchlorid (DDAC), Benzalkoniumchlorid (BAC), Dioctyldimethylammoniumchlorid, N,N-Dialkyl-N,N-dimethylammoniumcarbonat und Mecetroniumetilsulfat (MES) oder einer Mischung davon.

8. Mittel gemäß einem der vorhergehenden Ansprüche, **dadurch gekennzeichnet, dass** als Vitamin, dessen Salze oder saure Derivate mindestens eine Verbindung ausgewählt ist aus der Gruppe der Vitamine A, B, E und C.

9. Mittel gemäß einem der vorhergehenden Ansprüche, **dadurch gekennzeichnet, dass** das Metallion ein Ion eines Metalls der vierten Periode, der Nebengruppen I., II. oder VIII. des periodischen System der Elemente ist, vorzugsweise ein Eisen-, Kupfer- oder Zinkion.

10. Mittel gemäß einem der vorhergehenden Ansprüche, **dadurch gekennzeichnet, dass** als oberflächenaktive Substanz mindestens eine Verbindung, ausgewählt aus der Gruppe bestehend aus anionischen, nichtionischen, amphoteren oder kationischen Tensiden oder eine Mischung hiervon, enthalten ist.

11. Mittel gemäß einem der vorhergehenden Ansprüche, **dadurch gekennzeichnet, dass** das Mittel einen pH-Wert im Bereich von 0,5 bis 8,5 aufweist.

12. Mittel gemäß einem der vorhergehenden Ansprüche, **dadurch gekennzeichnet, dass** das Vitamin in Mengen von 0,1 mM bis 1000 mM enthalten ist.

13. Mittel gemäß einem der vorhergehenden Ansprüche, **dadurch gekennzeichnet, dass** das Metallion in Mengen von 0,01 mM bis 100 mM enthalten ist.

14. Mittel gemäß einem der vorhergehenden Ansprüche, **dadurch gekennzeichnet, dass** die oberflächenaktive Substanz in Mengen von 0,01 bis 35 Gew.-% der Gesamtlösung enthalten ist.

15. Verwendung eines Mittels gemäß einem der vorhergehenden Ansprüche zur Dekontamination und Desinfektion von belebten Oberflächen, vorzugsweise zur Desinfektion und Dekontamination von Haut, Haaren, Schleimhäuten, Wunden und Händen.

16. Verwendung eines Mittels gemäß einem der Ansprüche 1 bis 14 zur Desinfektion und Dekontamination unbelebter Oberflächen, vorzugsweise zur Desinfektion von Instrumenten oder Oberflächen.

17. Verwendung einer Zusammensetzung gemäß einem der Ansprüche 1 bis 14 zur Herstellung eines Mittels zum Biofilmabbau.
